(19) Europäisches Patentamt
European Patent Office
Office européen des brevets

(11) Numéro de publication : **0 003 708**
**B1**

(12) **FASCICULE DE BREVET EUROPÉEN**

(45) Date de publication du fascicule du brevet : **19.08.81**

(51) Int. Cl.³ : **C 07 C 33/02**, C 07 C 29/00, C 07 C 29/10

(21) Numéro de dépôt : **79400092.7**

(22) Date de dépôt : **13.02.79**

(54) **Nouveau procédé de préparation stéréosélectif de phéromones sexuelles et phéromones sexuelles obtenues.**

(30) Priorité : **15.02.78 FR 7804257**

(43) Date de publication de la demande : **22.08.79 (Bulletin 79/17)**

(45) Mention de la délivrance du brevet : **19.08.81 Bulletin 81/33**

(84) Etats contractants désignés : **BE CH DE GB IT LU NL**

(56) Documents cités :
**TETRAHEDRON LETTERS, Vol. 18, avril 1975 Oxford-New York (USA)**
**BRAUNSCHWEIG : « Synthèse d'enynes et de diènes conjugués à l'aide d'organocuivreux vinyliques. Application à la synthèse du bombykol » (J.F. NORMANT, A. COMMERCON, J. VILLIERAS) Pages 1 465-1 468**

**SYNTHESIS. International Journal of Methods in Synthetic Organic Chemistry 1978, n° 5, May, Stuttgart**
**D. SAMAIN, C. DESCOINS : « A short Stereoselective Synthesis of 8E, 10E-Dodecadien-1-ol. The Sex Pheromone of the codling Moth, Laspeyresia Pomonella, L » Pages 388-389**

(73) Titulaire : **Etablissement public dit: INSTITUT NATIONAL DE LA RECHERCHE AGRONOMIQUE**
**149, rue de Grenelle**
**F-75341 Paris Cedex 07 (FR)**

(72) Inventeur : **Samain, Daniel**
**9, Avenue de Verdun**
**F-60000 Compiègne (FR)**
Inventeur : **Kunesch, Gerhard**
**7, Allée Clément Marot**
**F-91400 Orsay (FR)**
Inventeur : **Descoins, Charles**
**27, Square des Platanes**
**F-78870 Bailly (FR)**

(74) Mandataire : **Nony, Michel**
**Cabinet Nony 29, rue Cambacérès**
**F-75008 Paris (FR)**

**EP 0 003 708 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 17, rue du Louvre, 75001 Paris, France

Nouveau procédé de préparation stéréosélectif de phéromones sexuelles et phéromones sexuelles obtenues

La présente invention a pour objet un nouveau procédé de préparation stéréosélectif de phéromones sexuelles et en particulier de phéromones ayant une structure alcool diénique conjugué de configuration stéréochimique trans-trans (E-E).

Les phéromones sexuelles de synthèse ont fait l'objet ces derniers temps de travaux ayant permis de démontrer le très grand intérêt que représente ces substances dans la lutte contre les principaux lépidoptères déprédateurs des cultures fruitières.

On peut à cet égard citer les travaux de H. AUDEMARD et coll. (Revue de Zoologie Agricole et de Pathologie Végétale 76, 15-24 1977) qui se rapportent à la lutte contre le Carpocapse (Laspeyresia Pomonella L.) à l'aide d'une phéromone sexuelle de synthèse connue sous la dénomination commerciale de Codlémone (ou dodécadiène-8E, 10E ol-1) dans les vergers de pommiers par la méthode dite de « confusion des mâles ».

On rappelera que le principe de cette méthode consiste à diffuser en permanence une certaine quantité de phéromones dans la parcelle du verger à contrôler de telle sorte que l'atmosphère soit uniformément imprégnée des vapeurs de cette substance. Les insectes mâles évoluant dans un tel environnement incapables de localiser les appels odorants de leurs femelles ne peuvent dès lors venir les féconder et, de ce fait, il en résulte une suppression des attaques des fruits et une diminution de la population des insectes de génération en génération, voire son élimination totale.

Cette méthode de lutte contre les déprédateurs des cultures fruitières effectuée jusqu'à présent à titre expérimental a permis de montrer tout l'intérêt que présentaient les phéromones sexuelles attractives puisque dans le cas du Carpocapse il a été constaté que les attaques touchaient moins de 0,5 % des fruits.

Cette méthode de lutte n'a pu toutefois trouver jusqu'à présent une généralisation car les méthodes de synthèse connues des phéromones sexuelles des principaux déprédateurs sont longues c'est-à-dire comprennent de nombreuses étapes et par ailleurs les produits obtenus ne présentent pas toujours la pureté stéréochimique voulue.

Il s'en suit que de nombreuses purifications sont alors nécessaires ce qui inévitablement conduit à un prix de revient de ces substances relativement élevé.

On comprend donc que de ce fait les phéromones sexuelles ne fassent pas encore partie de l'arsenal des moyens de lutte des arboriculteurs.

Les différentes phéromones sexuelles isolées, identifiées et synthétisées jusqu'à ce jour ont permis de montrer qu'il s'agissait de molécules de faible poids moléculaire à chaîne linéaire hydrocarbonée insaturée, généralement mono ou bi-insaturée, possédant une fonction alcool, acétate ou aldéhyde.

Les recherches effectuées ont par ailleurs démontré l'importance capitale que jouait la stéréochimie cis ou trans (Z ou E) de la ou des insaturations dans l'action de confusion des phéromones sexuelles de synthèse.

En effet on a constaté que le facteur stéréochimique pouvait à lui seul assurer la spécificité du signal vis-à-vis des mâles.

Par ailleurs on a remarqué que lorsque l'on utilisait des phéromones sexuelles de synthèse contaminées par un ou plusieurs autres isomères même en quantité relativement faible l'action de confusion de l'isomère actif était considérablement réduite voir totalement inhibée.

Il importe donc que les méthodes de synthèse des phéromones sexuelles soient totalement stéréosélectives c'est-à-dire ne conduisent pas par des réactions secondaires d'isomérisation à des mélanges d'isomères.

Par ailleurs ces synthèses ne doivent comporter que peu d'étapes de façon à obtenir de bons rendements et être réalisables à partir de produits intermédiaires facilement accessibles et en particulier à partir de produits du commerce.

La présente invention se rapporte à cet égard à un nouveau procédé de préparation stéréosélectif de phéromones sexuelles de grande pureté stéréochimique et en particulier de phéromones ayant une structure alcool diénique conjugué de configuration stéréochimique trans-trans (E-E) correspondant à la formule générale suivante :

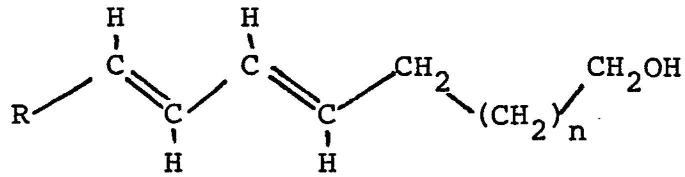

(I)

dans laquelle :

R représente un radical alkyl saturé linéaire ayant de 1 à 4 atomes de carbone et n est un nombre entier de 1 à 5.

Diverses synthèses de composés de ce type et notamment la Codlémone, $R = CH_3$ et $n = 5$

(dodécadiène-8E, 10E ol-1) ont déjà été proposées dans la littérature.

On peut en particulier citer parmi les synthèses de la Codlémone celle de la Société Zoecon Corporation décrite dans le brevet américain N° 3.825.607, et celle de K. MORI décrite dans Tetrahedron vol. 30, p. 1 807-3 810, (1974).

Dans ces synthèses l'étape principale consiste soit en un couplage entre une unité en $C_7$ (bromo-1 heptadiène-3E, 5E) et une unité en $C_5$ (magnésien du [chloro-5' pentyloxy]-2 tetrahydropyranne) soit un couplage entre une unité en $C_6$ (bromo-1 hexadiène-2E, 4E) et une autre unité en $C_6$ (magnésien du [chloro-6' hexyloxy]-2 tetrahydropyranne).

Au cours de la réaction de couplage entre l'unité en $C_7$ et l'unité en $C_5$ il se produit une isomérisation partielle conduisant à un mélange d'isomères dont le trans-trans est cependant prépondérant.

De même au cours de la réaction de couplage entre l'unité en $C_6$ et l'autre unité en $C_6$ intervient également une isomérisation accompagnée d'une transposition allylique.

L'obtention de l'isomère trans-trans à l'état stéréochimiquement pur nécessite donc dans ces deux synthèses des purifications poussées ce qui a pour effet d'obtenir la Codlémone avec des rendements relativement faibles.

Par ailleurs les méthodes de purification facilement réalisables à l'échelle du laboratoire sont difficilement transposables au stade industriel.

Après de longues recherches on a constaté de façon tout-à-fait surprenante et inattendue que les phéromones sexuelles de synthèse de formule (I) ci-dessus pouvaient être obtenues de façon simple et rapide avec d'une part un excellent rendement et d'autre part avec une grande pureté stéréochimique en réalisant le couplage entre un dérivé magnésien et un acétate, tosylate ou mésylate d'un alcool diénique conjugué de configuration stéréochimique trans-trans dans des conditions particulières de réaction notamment en ce qui concerne la température de couplage et par ailleurs les proportions des réactifs mis en jeu.

Les essais réalisés ont en effet permis de montrer que l'excellent rendement et la rétention de la configuration stéréochimique n'était possible dans ce type de couplage que si le précurseur diénique était uniquement de configuration trans-trans.

En effet on a constaté que si le précurseur diénique était cis-trans (Z,E), cis-cis (Z,Z) ou trans-cis (E,Z) il n'y avait pas rétention de la stéréochimie et de la sorte la réaction conduisait à l'obtention d'un mélange d'isomères.

On doit cependant remarquer que J. NORMANT et al. Tétrahédron Letters volume n° 18, pages 1 465-1 468, 1975 ont décrit avoir réalisé la synthèse du bombykol (héxadécadiène-10 E, 12 Z ol-1) avec rétention de la stéréochimie en utilisant ce type de couplage selon des conditions opératoires différentes sans cependant donner d'indication précise sur la pureté stéréochimique du produit obtenu.

La présente invention a pour objet un nouveau procédé de préparation stéréosélectif de phéromones sexuelles ayant une structure alcool diénique conjugué de configuration stéréochimique trans-trans (E-E) correspondant à la formule générale suivante :

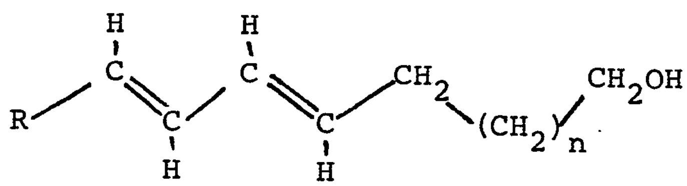

(I)

dans laquelle :

R représente un radical alkyl saturé linéaire ayant de 1 à 4 atomes de carbone et n est un nombre entier de 1 à 5, caractérisé par le fait que dans un premier temps on prépare en milieu tétrahydrofuranne un dérivé magnésien correspondant à la formule suivante :

$$XMg—(CH_2)_n—CH_2OP \qquad (II)$$

dans laquelle :

X représente un halogène de préférence Br ou Cl, et P est un groupe protecteur, et que dans un deuxième temps on ajoute à une température comprise entre 0 et −20 °C ledit dérivé magnésien en excès à une solution tétrahydrofurannique contenant un catalyseur au cuivre et un composé diénique trans-trans correspondant à la formule suivante :

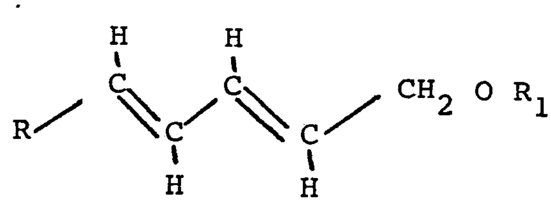

(III)

dans laquelle :

R a la même signification que ci-dessus et $R_1$ représente un radical $-COCH_3$, $-SO_2CH_3$ et $-SO_2C_6H_4CH_3$ et qu'enfin on soumet le produit de réaction à une hydrolyse pour libérer le groupe protecteur.

Ce procédé de préparation permet en particulier la synthèse des composés de formule (I) dans lesquels :

1) $R = CH_3$ et $n = 5$, dodécadiène 8E, 10E, ol-1 (Codlémone),

2) $R = C_2H_5$ et $n = 4$, dodécadiène-7E, 9E ol-1,

3) $R = C_3H_7$ et $n = 3$, dodécadiène-6E, 8E ol-1, et

4) $R = C_4H_9$ et $n = 4$, tétradécadiène-7E, 9E ol-1,

Ces composés sont obtenus par le procédé selon l'invention avec un rendement supérieur ou égal à 60 % et avec une pureté stéréochimique supérieure ou égale à 98 % en isomère trans-trans.

Les dérivés magnésiens de formule (II) sont facilement accessibles à partir des halogénoalkanols W-W' correspondants dont la fonction OH a été au préalable protégée à l'aide d'un groupe protecteur.

Parmi les halogénoalkanols W-W' on peut utiliser ceux disponibles dans le commerce et notamment le chloro ou bromo-3 propanol-1, le chloro ou bromo-4 butanol-1, le chloro ou bromo-5 pentanol-1, le chloro ou bromo-6 hexanol-1 et le chloro ou bromo-7 heptanol-1.

Comme groupe protecteur de la fonction OH des halogénoalkanols on peut utiliser des groupes formant des liaisons du type acétal tels que des groupes tétrahydropyrannyl et tétrahydrofurannyl ainsi que certains groupes silyl tels que triméthylsilyl et triéthylsilyl.

D'autres groupes protecteurs peuvent également être utilisés tels que triphénylméthyl, diphénylméthyl, benzyl et t-butyl.

Selon une forme particulière de réalisation on préfère utiliser selon l'invention comme groupe protecteur des groupes tétrahydrofurannyl ou tétrahydropyrannyl.

Les composés de formule (II) sont facilement obtenus en faisant réagir les halogénoalkanols W-W' correspondants avec du dihydrofuranne ou dihydropyranne en présence d'un catalyseur acide et en particulier d'un acide fort tel que de l'acide chlorhydrique, de l'acide p-toluène sulfonique, de l'acide sulfurique ou de l'acide méthanesulfonique.

Les composés de formule (III) sont également aisément accessibles à partir des alcools diéniques-2E, 4E correspondants dans les conditions classiques de formation des dérivés acétoxy, mésyl et tosyl.

Selon un mode préféré de l'invention on utilise les dérivés acétoxy obtenus par réaction d'acétylation des alcools diéniques-2E, 4E par l'anhydride acétique dans la pyridine.

Parmi les alcools diéniques-2E, 4E conduisant aux composés de formule (III) on peut en particulier citer l'hexadiène-2E, 4E ol-1, l'heptadiène-2E, 4E, ol-1, l'octadiène-2E, 4E ol-1 et le nonadiène-2E, 4E ol-1.

Ces alcools diéniques sont pour la plupart disponibles dans le commerce et notamment vendus par la Société ORIL.

Selon l'invention le catalyseur au cuivre peut être de l'iodure cuivreux, du chlorure ou du bromure cuivreux ou du dilithium tétrachlorocuprate ($Li_2\ Cu\ Cl_4$).

Selon une forme préférée de l'invention on utilise ce dernier catalyseur obtenu à partir du chlorure de lithium et du chlorure cuivrique dans un rapport molaire de 2:1 selon la méthode décrite par Tamura et coll. Synthesis, Juillet 1971 page 303.

On utilise en général selon l'invention une quantité de catalyseur comprise entre 0,01 et 0,06 mole et de préférence environ 0,04 mole par mole de composé de formule (III).

Comme indiqué précédemment la température d'addition du magnésien revêt une grande importance et doit être effectuée dans la gamme comprise entre 0 et −20 °C et de préférence à environ −10 °C.

Après addition du magnésien à la solution tétrahydrofurannique du composé de formule (III), la concentration finale des réactifs dans le tétrahydrofuranne doit être comprise entre environ 0,1N et 0,8N et de préférence d'environ 0,5N. On laisse ensuite se poursuivre la réaction de couplage à température ambiante pendant une durée comprise entre 1 et 5 heures et de préférence pendant environ 3 heures.

Comme indiqué ci-dessus, il importe dans le but d'obtenir un excellent rendement d'utiliser le magnésien de formule (II) en excès par rapport au composé de formule (III), cet excès devant être d'au moins 0,2 mole de magnésien par rapport à la quantité molaire de composé de formule (III) et de préférence comprise entre 0,25 à 0,6 mole.

Après la fin de la réaction de couplage le mélange réactionnel est hydrolysé à froid à une température comprise entre environ 0° et −20 °C et de préférence à environ −10 °C par la quantité stoéchiométrique d'une solution saturé de chlorure d'ammonium.

Le composé intermédiaire formé de formule :

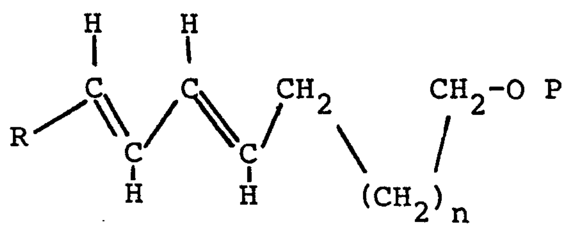

(IV)

dans laquelle : R, n et P ont les mêmes significations que ci-dessus, est ensuite soumis, après évaporation des solvants de réaction et d'extraction, à une hydrolyse en vue de libérer le groupe protecteur et conduire au composé de formule (I).

La nature de l'agent hydrolysant et les conditions d'hydrolyse dépendent de la nature du groupe protecteur.

Lorsque le groupe protecteur est un groupe tétrahydropyrannyl ou tétrahydrofurannyl on utilise de préférence de l'acide p-toluène sulfonique en milieu alcoolique (méthanol) et la température d'hydrolyse est généralement comprise entre environ 20 à 60 °C.

Lorsque le groupe protecteur est par contre un groupe silyl celui-ci peut être éliminé selon la technique décrite dans J. Am. Chem. Soc. 74.3024 (1952).

Après cette étape d'hydrolyse le résidu, après l'évaporation des solvants d'extraction, est soumis à une distillation sous vide en vue d'obtenir le composé de formule (I) sous forme purifiée.

Certains des alcools de formule (I) obtenus par le procédé selon l'invention peuvent se présenter sous forme cristallisée et il est donc possible de les obtenir également sous forme purifiée par cristallisation dans un solvant approprié par exemple dans un hydrocarbure tel que du pentane ou de l'hexane.

Les alcools diéniques conjugués de formule (I) obtenus par le procédé selon l'invention peuvent si nécessaire subir d'autres transformations et notamment être transformés en acétates, propionates, butyrates et isobutyrates selon les méthodes conventionnelles de même qu'en aldéhydes correspondants par réaction d'oxydation à l'aide de perchlorate de pyridinium.

Ces dérivés sont en effet également des attractifs sexuels pour de nombreux lépidoptères ravageurs des cultures.

Dans le but de montrer que l'excellente stéréosélectivité du procédé selon l'invention n'est possible qu'au départ de composés de formule (IV) de stéréochimie trans-trans (E-E) on a réalisé dans les mêmes conditions opératoires le couplage suivant :

$$CH_3\ CH_2\ (CH{=}CH)_2\ CH_2\ OCOCH_3 \ \text{(a)}$$
$$+$$
$$MgCl - (CH_2)_4 - CH_2\ OThP \ \text{(b)}$$
$$\xrightarrow[\text{2) } H^+]{\text{1) Cu}} CH_3CH_2\ (CH{=}CH)_2-(CH_2)_5-CH_2OH$$

(ThP = Tétrahydropyrannyl)

au départ des isomères 2-E 4-E, 2-E 4-Z, 2-Z 4-Z et 2-Z 4-E de l'acétoxy-1 héptadiène-2,4 (a).

Après hydrolyse et distillation sous vide, la chromatographie en phase vapeur des 4 préparations a donné les résultats suivants :

| Stéréochimie de l'acétoxy-1 heptadiène-2,4 de départ (pureté ≥ à 98 %) | Température d'addition du magnésien (b) | Excès du magnésien (mole) | Dodécadiène-7, 9 ol-1 obtenus (%) | | | |
|---|---|---|---|---|---|---|
| | | | Isomère 7Z,9E | Isomère 7E,9Z | Isomère 7Z,9Z | Isomère 7E,9E |
| 2E,4E | – 10 °C | 0,5 | 0 | 0 | 0 | 100 |
| 2E,4Z | – 10 °C | 0,5 | 0 | 75 | 0 | 25 |
| 2Z,4Z | – 10 °C | 0,5 | 3 | 27 | 60 | 10 |
| 2Z,4E | – 10 °C | 0,5 | 61 | 2 | 1 | 36 |

Comme on peut le constater seul le couplage réalisé à partir de l'acétoxy-1 heptadiène-2E, 4-E a permis de conduire à une rétention de la stéréochimie dans le Dodécadiène-7,9 ol-1 obtenu.

On va maintenant donner à titre d'illustration et sans aucun caractère limitatif plusieurs exemples de préparation de phéromones sexuelles de formule (I) par le procédé selon l'invention.

## Exemples

### Exemple 1

*Préparation de l'Acétoxy-1 héxadiène-2E, 4E* : $C_8\ H_{12}O_2 = 140$ (formule III, $R = CH_3$ $R_1 = -COCH_3$)

1 kg d'héxadiène-2E, 4E ol-1 (pureté stéréochimique 98 % E,E) sont dissous dans 2,8 litres de pyridine (distillée au préalable sur potasse). La solution est refroidie en dessous de +10° et on ajoute en 2h,

2,8 litres d'anhydride acétique. Après la fin de l'addition on laisse remonter la température à +20° pendant 3h. On verse ensuite le mélange réactionnel sur 10 kg de glace pilée et on extrait 2 fois avec 2 litres de pentane technique. La phase organique est successivement lavée avec de l'eau (500 $cm^3$), de l'acide sulfurique 2N (500 $cm^3$), une solution saturée de bicarbonate de sodium (500 $cm^3$), de nouveau à l'eau et enfin avec une solution saturée de chlorure de sodium (500 $cm^3$). Après séchage sur sulfate de magnésium et évaporation du pentane, la distillation du résidu donne 1,633 kg d'acétate (81 %) $Eb_{20}$ = 78 °C.

Selon le même mode opératoire on a préparé l'acétoxy-1 heptadiène-2E, 4E à partir de l'héptadiène-2E, 4E ol-1 avec un rendement de 80 % $Eb_{20}$ = 85 °C.

Exemple 2

*Préparation du (chloro-6' héxyloxy)-2 tétrahydropyranne* : $C_{11}H_{21}O_2Cl$ = 220,5

3 kg de chloro-6 hexanol-1 du commerce sont coulés dans un réacteur de 10 litres et on ajoute 7,5 $cm^3$ d'acide chlorhydrique concentré. On refroidit le mélange réactionnel en dessous de +10° par une saumure (glace pilée + méthanol) et on ajoute en 3 h par portions de 100 $cm^3$ à la fois 2 kg 125 de dihydropyranne fraîchement distillé. Après la fin de l'addition on laisse 3 h à température ambiante, dilue avec 2 litres d'éther et lave avec le mélange réactionnel successivement avec : 500 ml de soude 2N, 2 litres d'eau et 2 litres d'une solution saturée de chlorure de sodium.

On sèche sur sulfate de magnésium, évapore l'éther et distille le résidu sous vide.

On obtient : une fraction intermédiaire de 400 g $Eb_{0,1}$ = 40-106° et une fraction principale correspondant au produit cherché de 4 kg 311 (Rdt 91 %) ; $Eb_{0,1}$ = 106-110°.

Ce produit est caractérisé par son spectre I.R.

IR (film) ; bandes à : 2 930, 2 860, 1 460, 1 450, 1 440, 1 350,1 200, 1 130, 1 120, 1 075, 1 030, 900, 865, 810 $cm^{-1}$.

Exemple 3

*Préparation du tétrahydropyrannyl éther du dodécadiène-8E, 10E, ol-1.* (formule IV, R = $CH_3$, n = 5 et P = tétrahydropyrannyl)

(Les réactions a) et b) suivantes ont été conduites sous Argon).

a) Préparation du magnésien du (chloro-6' héxyloxy)-2 tétrahydropyranne.

Dans un réacteur de 4 litres muni d'une agitation mécanique d'une ampoule à brome et d'un réfrigérant ascendant, on introduit 60 g de magnésien en copeaux qu'on recouvre de 100 ml de tétrahydrofuranne (THF). On ajoute d'un seul coup 10 g de (chloro-6' héxyloxy)-2 tétrahydropyranne obtenu selon l'exemple 2 et on porte le mélange réactionnel à reflux. Lorsque l'ébullition s'amorce, on ajoute 5 $cm^3$ de dibromoéthane ; il se produit alors une réaction très violente avec attaque du magnésium et noircissement du mélange réactionnel. On démarre alors l'agitation et tout en maintenant le reflux on introduit en 2 h, goutte à goutte, une solution de 425 g de (chloro-6' hexyloxy)-2 tétrahydropyranne dans 1,5 litre de tétrahydrofuranne.

Après la fin de l'addition on laisse encore à reflux pendant 3 h et laisse reposer une nuit à température ambiante.

b) réaction de couplage entre l'acétoxy-1 hexadiène-2E, 4E préparé selon l'exemple 1 et le magnésien précédent :

Dans un réacteur de 6 litres muni d'une agitation mécanique, d'un thermomètre, on siphone une solution de 180 g d'acétoxy-1 hexadiène-2E, 4E dans 1,5 litre de tétrahydrofuranne.

On refroidit le mélange réactionnel à −10° et on y ajoute (par siphonage) une solution de 4,3 g de chlorure de lithium anhydre et de 6,8 g de chlorure cuivrique anhydre dans 90 $cm^3$ de tétrahydrofuranne. Le mélange réactionnel est rouge orangé.

On ajoute alors en 1 h 30 environ la solution magnésienne précédente, en veillant à ce que la température ne dépasse pas −10 °C. Au début de l'addition le mélange réactionnel prend une coloration vert clair, puis se décolore presque complétement et vire enfin au violet noir.

Après la fin de l'addition on laisse 3 h à 0 °C, refroidit de nouveau à −10 °C et hydrolyse avec une solution saturée de chlorure d'ammonium, sous violente agitation, jusqu'à ce que la solution se décolore et qu'un abondant précipité noir et visqueux se dépose au fond du réacteur. On décante le surnageant et triture le résidu avec 3 fois 200 $cm^3$ d'éther. Les phases organiques rassemblées sont lavées successivement avec une solution saturée de chlorure d'ammonium (500 $cm^3$ ; 2 fois) puis avec une solution saturée de chlorure de sodium (500 $cm^3$ : 2 fois).

Les phases organiques sont ensuite transférées directement dans un évaporateur.

Après évaporation du solvant sous vide partiel (50 mm), on obtient 470 g de résidu, constitué essentiellement par le tétrahydropyrannyl éther du dodécadiène-8E,10E, ol-1.

Exemple 4

*Préparation du dodécadiène-8E, 10E ol-1* : (formule I, R = $CH_3$ et n = 5)

Le résidu obtenu à l'exemple 3 est dissous dans 2,5 litres de méthanol et 250 $cm^3$ d'eau et on ajoute 42 g d'acide p-toluène sulfonique. On chauffe à 60° pendant 3 h et laisse une nuit à température ambiante. On évapore la majorité du méthanol sous vide reprend le résidu dans 2 fois son volume d'éther et le lave successivement à l'eau (300 $cm^3$), avec une solution saturée de bicarbonate de sodium (300 $cm^3$), de nouveau à l'eau et enfin avec une solution saturée de chlorure de sodium.

On sèche alors sur sulfate de magnésium.

Après évaporation du solvant la distillation du résidu (270 g) donne :

59 g de produit de tête $Eb_{0,1}$ = 40-98°, 140 g de dodécadiène-8E,10E ol-1 Eb 0,1 = 98-100° qui cristallise en cours de distillation et 71 g de résidu constitué essentiellement de dodécane-diol.

Le rendement en dodécadiène-8E, 10E, ol-1 distillé et cristallisé est de 62,5 %, calculé sur l'acétoxy-1 hexadiène 2E, 4E mis en œuvre.

La pureté stéréochimique de ce produit est de 99,5 % évaluée par chromatographie en phase gazeuse (CPV) :

colonne 5 m inox 1/8" 3 % F.F.A.P/chrom G.H.P. 80/100 - 190 °C :

il est caractérisé :

(i) *par son spectre I.R.* ($cm^{-1}$)

bandes à 3 370 (OH associé) ; 1 615 (−C=C) ; 1 055 (OH primaire) ; 980(−CH=CH−, E, conjugué). L'absence de bande à 950 $cm^{-1}$ dénote l'absence d'isomère 8Z, 10E.

(ii) *par son spectre R.M.N.* (§, ppm, 250 Mhz, $CDCl_3$) 1,3, S. large 8H, $4CH_2$ ; 1, 54, quint, 2H, $-CH_2$ en $C_1$ ; 7, d, 3H, $-CH_{-3}$ ; 2, 04, q, 2H, = $-CH_{-2}$ ; 3, 6, t, 2H, $-CH_{-2}$ OH ; 5, 54, m, 2H, $H_{11}$ et $H_8$ ; 5,96, 2H, $H_9$ et $H_{10}$.

(iii) *par son spectre de masse* : $M^+$ = 182 ; fragments à 164 ($M^+ - H_2O$) ; 135 ($M^+ - (H_2O + C_2H_5)$), 121, 107, 81, 79, 68, 67, 55, 42, 32.

et (iv) par son point de fusion : F = 29-30°.

— Selon une variante du procédé précédent il est possible avant d'effectuer la distillation sous vide d'isoler le dodécanediol du produit brut d'hydrolyse en le reprenant dans 3 fois son volume de pentane et en le laissant à température ambiante pendant 3 heures. En effet dans ces conditions le dodécanediol précipite et peut être éliminé par essorage sous vide.

Exemple 5

*Préparation du dodécadiène-8E, 10E ol-1* (formule I, R = $CH_3$ n = 5).

Selon les mêmes conditions opératoires que celles décrites aux exemples 3 et 4 ci-dessus on a réalisé la synthèse de 1,100 kg de dodécadiène-8E, 10E ol-1 à partir des quantités suivantes de solvants et de réactifs :

3,360 kg de (chloro-6' hexyloxy)-2 tétrahydropyranne
480 g de magnésium
1,440 kg d'acétoxy-1 hexadiène-2E, 4E
35 g de chlorure de lithium anhydre
55 g de chlorure cuivrique anhydre
29 litres de T.H.F., 15 litres de méthanol
300 g d'acide p-toluène sulfonique.

Dans cette préparation le rendement en dodécadiène-8E, 10E ol-1 distillé et cristallisé à été de 60 %.

Si on le désire le dodécadiène-8E, 10E ol-1 peut être obtenu sous forme analytiquement pur en procédant comme suit :

140 g de dodécadiène 8E, 10E ol-1 provenant de la réaction précédente sont dissous dans 280 $cm^3$ de pentane (qualité nanograde BYKE - MALLINCRODT). La solution est lentement refroidie jusqu'à −10 °C, temps pendant lequel le dodécadiène-8E, 10E ol-1 cristallise sous forme d'aiguilles blanches.

On essore à −10 °C et obtient 133 g de dodécadiène-8E, 10E ol-1 rigoureusement pur en C.P.V. (conditions analytiques précédentes) F = 32 °C.

Cette haute pureté n'est pas nécessaire pour mettre en œuvre la méthode de confusion des mâles dans les vergers.

Exemple 6

*Préparation de l'acétoxy-1 octadiène-2E,4E* (formule III, R = $C_3H_7$ et $R_1$ = $-COCH_3$).

L'octadiène-2E, 4E ol-1 commercial est d'abord recristallisé dans du pentane à froid.

10 g (0,08 mole) d'alcool recristallisé sont alors acétylés par 16,3 g (0,16 mole) d'anhydride acétique en solution dans 20 ml de pyridine. Après 3 h à température ambiante et hydrolyse à la manière habituelle, on obtient : 12 g (90 %) d'acétate correspondant $Eb_{0,1}$ = 50° C.P.V. : produit unique sur colonne 5 % E.C.N.S.S.M. sur Gas Chrom Q 100:120 mesh, 3 m inox 1/8, 130°. I.R. ($cm^{-1}$, film) 1 740 (C=O ester), −C=C− 1 660, −CH=CH− E conjugués 990 (s) et 945 (w).

Exemple 7

*Préparation du dodécadiène-6E, 8E ol-1* (formule I, R = $C_3H_7$ et n = 3) *et de son dérivé acétylé* :

A l'acétate obtenu à l'exemple 6 (4g, 0,023 mole), en présence de 8 ml d'une solution de dilithium tétrachlorocuprate préparé comme précédemment on ajoute à −10° un excès du magnésien du (chloro-4' butyloxy)-2 tétrahydropyranne (35 $cm^3$ d'une solution N dans le T.H.F.) dans les mêmes conditions de réaction que celles décrites à l'exemple 3 b) ci-dessus.

Après hydrolyse et évaporation du solvant, le résidu est traité par l'acide p-toluène sulfonique dans le méthanol selon les mêmes conditions qu'à l'exemple 4 ce qui permet d'obtenir le dodécadiène-6E, 8E ol-1.

Ce dernier sans être purifié est traité par l'anhydride acétique (3,36 g, 0,033 mole) à la manière habituelle et fournit 3,3 g (64 %) d'acétoxy-1 dodécadiène-6E-8E, Eb 0,1 = 88°.

C.P.V. : Produit unique sur 3 % F.F.A.P, Gas Chrom G, 5 m inox 1/8, 190°

I.R. (cm−1 film) 1 740 (C=O ester, 985 (s) et 955 (w) −CH=CH− E conjugués.

Exemple 8

*Préparation de l'acétoxy-1 nonadiène-2E, 4E* (formule III, R = $C_4H_9$ et $R_1$ = $COCH_3$)

Le nonadiène-2E, 4E ol-1 commercial est d'abord recristallisé dans du pentane.

10 g (0,071 mole) d'alcool sont alors acétylés par 14,4 g (0,142 mole) d'anhydride acétique en solution dans 20 ml de pyridine. On obtient après les traitements habituels 11,5 g (87 %) d'acétate $Eb_{0,1}$ = 70°.

C.P.V. : Produit unique, 5 % E.C.N.S.S.M. sur Gas Chrom Q 100/120 mesh, 3 m inox 1/8, 140°.

I.R. (film, $Cm^{-1}$) 1 740 (C = O ester) ; 1 660 (C = C) ; 950 (s) et 945 (w) (−CH=CH−, E conjugués).

Exemple 9

*Préparation du tétradécadiène-7E, 9E, ol-1* : (formule I, R = $C_4H_9$ et n = 4) *et de son dérivé acétylé.*

A l'acétate obtenu à l'exemple 8 (5 g, 0,027 mole) en présence de 10 ml d'une solution de dilithium tétrachlorocuprate on ajoute à −10° par une solution N du magnésien du (chloro-5' pentyloxy)-2 tétrahydropyranne dans le T.H.F. (40,5 $cm^3$) dans les mêmes conditions que celles décrites à l'exemple 3 b) ci-dessus.

Après hydrolyse et évaporation du solvant, le résidu est traité par l'acide p-toluène sulfonique dans le méthanol selon les mêmes conditions qu'à l'exemple 4 ce qui permet d'obtenir le tétradécadiène-7E, 9E ol-1. Ce dernier sans être purifié est traité par l'anhydride acétique (3,6 g, 0,036 mole) à la manière habituelle et fournit 4 g (60 %) d'acétoxy-1 tétradécadiène-7E, 9E $Eb_{0,1}$ = 105°.

C.P.V. : Produit unique, 3 % FFAP sur Gas Chrom G, 5 m inox 1/8, 200°

I.R. (film, $Cm^{-1}$) 1 740 (C=O ester) ; −CH=CH−, E, conjugués : 985 (s) et 955 (w).

**Revendications**

1. Procédé de préparation stéréosélectif de phéromones sexuelles ayant une structure alcool diénique conjugué de configuration stéréochimique trans-trans (E-E) correspondant à la formule suivante :

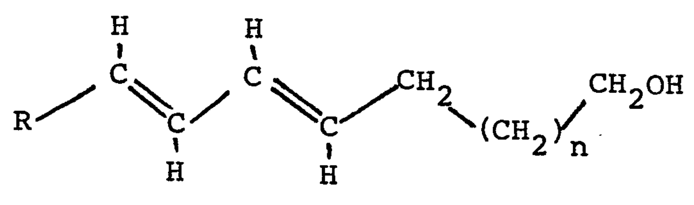

(I)

dans laquelle :

R représente un radical alkyl saturé linéaire ayant de 1 à 4 atomes de carbone et n est un nombre entier de 1 à 5, caractérisé par le fait que dans un premier temps on prépare en milieu tétrahydrofuranne un dérivé magnésien correspondant à la formule suivante :

$$XMg—(CH_2)_n—CH_2\ OP \qquad (II)$$

dans laquelle :

X représente un halogène de préférence Br ou Cl, et P est un groupe protecteur, et que dans un deuxième temps on ajoute à une température comprise entre 0 et −20 °C ledit dérivé magnésien en excès à une solution tétrahydrofurannique contenant un catalyseur au cuivre et un composé diénique trans-trans correspondant à la formule suivante :

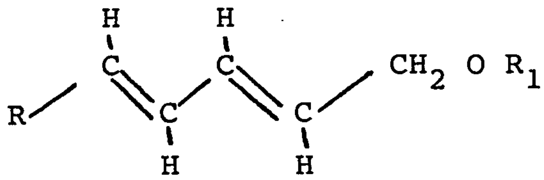

(III)

dans laquelle :

R a la même signification que ci-dessus et $R_1$ représente un radical $-COCH_3$, $-SO_2CH_3$ et $-SO_2C_6H_4CH_3$ et qu'enfin on soumet le produit de réaction à une hydrolyse pour libérer le groupe protecteur.

2. Procédé selon la revendication 1 caractérisé par le fait que le dérivé magnésien de formule (II) est obtenu à partir d'un halogénoalkanol W-W' dont la fonction OH a été au préalable protégée, ledit halogénoalkanol étant pris dans le groupe constitué par : le chloro ou bromo-3 pentanol-1, le chloro ou bromo-4 butanol-1, le chloro ou bromo-5 pentanol-1, le chloro ou bromo-6 hexanol-1 et le chloro ou bromo-7 heptanol-1.

3. Procédé selon l'une quelconque des revendications 1 et 2 caractérisé par le fait que le groupe protecteur P des composés de formule (II) est un groupe tétrahydropyrannyl, tétrahydrofurannyl, triméthylsilyl, triéthylsilyl triphénylméthyle, diphénylméthyl, benzyl ou t-butyl.

4. Procédé selon la revendication 1, caractérisé par le fait que le composé de formule (III) est obtenu par acétylation, mésylation ou tosylation d'un alcool diénique-2E, 4E pris dans le groupe constitué par : l'hexadiène-2E, 4E ol-1, l'heptadiène-2E, 4E ol-1, l'octadiène-2E, 4E ol-1 et le nonadiène-2E, 4E ol-1.

5. Procédé selon l'une quelconque des revendications précédentes caractérisé par le fait que le catalyseur au cuivre est de l'iodure cuivreux, du chlorure ou du bromure cuivreux ou du dilithium tétrachlorocuprate.

6. Procédé selon l'une quelconque des revendications précédentes caractérisé par le fait que la quantité de catalyseur est comprise entre 0,01 et 0,06 mole et de préférence d'environ 0,04 mole par mole de composé de formule (III).

7. Procédé selon l'une quelconque des revendications précédentes caractérisé par le fait que l'excès du dérivé magnésien de formule (II) est d'au moins 0,2 mole et de préférence compris entre 0,25 et 0,6 mole par rapport à la quantité molaire du composé de formule (III).

8. Procédé selon l'une quelconque des revendications précédentes caractérisé par le fait que la concentration finale des réactifs dans le tétrahydrofuranne est compris entre 0,1N et 0,8N et de préférence d'environ 0,5N.

9. Procédé selon l'une quelconque des revendications précédentes caractérisé par le fait que l'hydrolyse pour libérer le groupe protecteur est effectuée en milieu alcoolique en présence d'acide p-toluène sulfonique.

**Claims**

1. A process for the stereoselective preparation of sexual pheromones having an alcohol group and a conjugated diene structure of the trans-trans (E-E) stereochemical configuration corresponding to the following formula :

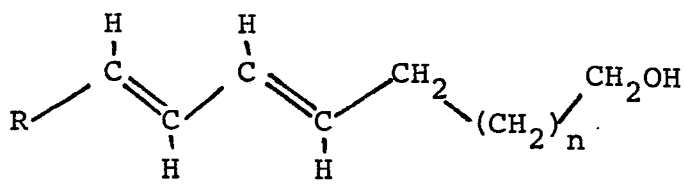

(I)

in which :

R represents a saturated linear alkyl radical having 1-4 carbon atoms and n is a number from 1 to 5, characterized by the fact that in the first stage a magnesium derivative is prepared in a tetrahydrofuran medium said derivative corresponding to the following formula :

$$XMg-(CH_2)_n-CH_2\ OP \qquad (II)$$

in which :

X represents halogen which is Br ou Cl, and P is a protective group and that in a second stage at a temperature ranging from 0 to $-20\,°C$, an excess of said magnesium derivative is added to a tetrahydrofuran solution containing a copper catalyst and a trans-trans dienic compound corresponding to the following formula :

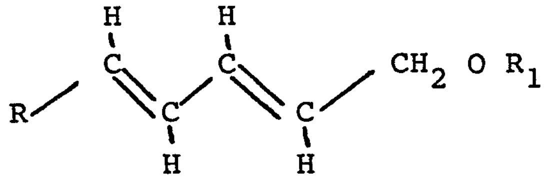

(III)

in which :

R has a same definition as above and $R_1$ is : $-COCH_3$, $-SO_2CH_3$ or $-SO_2C_6H_4CH_3$ and lastly, subjecting the product of the second stage to hydrolysis to liberate the protective group.

2. The process according to claim 1, characterized by the fact that the magnesium derivate of formula (II) is obtained starting from a halogenated alkanol W-W', the OH function of which has been protected, wherein said halogenated alkanol is selected from the group consisting of chloro-3 or bromo-3 pentanol-1, chloro-4 or bromo-4 butanol-1, chloro-5 or bromo-5 pentanol-1, chloro-6 or bromo-6 hexanol-1 and chloro-7 or bromo-7 heptanol-1.

3. The process according to anyone of claims 1 and 2, characterized by the fact that the protective group P in formula (II) is tetrahydropyrannyl, tetrahydrofurannyl, trimethylsilyl, triethylsilyl triphenylmethyl, diphenylmethyl, benzyl or t-butyl group.

4. The process according to claim 1, characterized by the fact that the compound of formula (III) is obtained by acetylation mesylation or tosylation of a dienic alcohol-2E, 4E, selected from the group consisting of hexadiene-2E, 4E ol-1 ; heptadiene-2E, 4E ol-1 ; octadiene-2E, 4E ol-1 and nonadiene-2E, 4E ol-1.

5. The process according to anyone of the preceeding claims characterized by the fact that the copper catalyst is copper iodide, copper chloride, copper bromide or dilithium tetrachlorocuprate.

6. The process according to anyone of the preceeding claims characterized by the fact that the quantity of the catalyst is from 0.01 to 0.06 mole, and preferably about 0.04 mole, per mole of the compound of formula (III)

7. The process according to anyone of the preceeding claims characterized by the fact that excess of the magnesium derivate of formula (II) is at least 0.2 mole and preferably from 0.25 to 0.6 mole based on the molar quantity of the compound of formula (III).

8. The process according to anyone of the preceeding claims characterized by the fact that final concentration of the reactants in the tetrahydrofuran is from 0.1N to 0.8N, and preferably is about 0.5N.

9. The process according to anyone of the preceeding claims characterized by the fact hydrolysis for liberating the protective group is effectuated in an alcoholic medium in the presence of p-toluene sulfonic acid.

**Ansprüche**

1. Verfahren zur stereoselektiven Herstellung von Pheromon-Sexuallockstoffen auf der Grundlage eines konjugierten Dien-alkohls mit trans-trans (E-E) stereochemischer Konfiguration, der nachstehenden allgemeinen Formel :

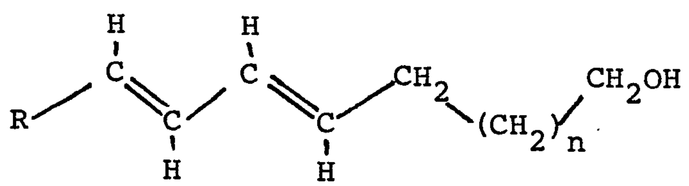

(I)

worin :

R einen linearen, gesättigten Alkylrest mit 1 bis 4 Kohlenstoffatomen darstellt und n eine ganze Zahl von 1 bis 5 bedeutet, dadurch gekennzeichnet, daß man in einer ersten Stufe in Tetrahydrofuran-Milieu eine Magnesiumderivat der nachstehenden Formel herstellt :

$$XMg—(CH_2)_n—CH_2\ OP \qquad (II)$$

worin :

X für ein Halogen, vorzugsweise für Br oder Cl steht, und P eine Schutzgruppe darstellt, und in einer zweiten Stufe bei einer Temperatur zwischen Null und 20 °C das genannte Magnesiumderivat im Überschuß zu einer Tetrahydrofuranlösung, enthaltend einen Kupferkatalysator und eine trans-trans Dienverbindung der nachstehenden Formel zugibt :

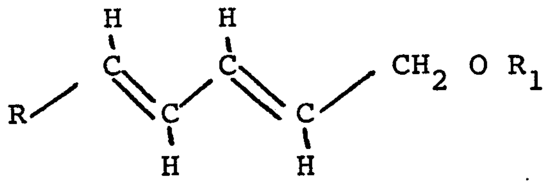

(III)

worin :

R dieselbe Bedeutung wie zuvor besitzt und $R_1$ einen Rest : $-COCH_3$, $-SO_2CH_3$ und $-SO_2C_6H_4CH_3$ bedeutet, und das Reaktionsprodukt anschließend hydrolysiert, um die Schutzgruppe freizusetzen.

2. Verfahren nach Anspruch 1, dadurch gekennzeichnet, daß das Magnesiumderivat der Formel (II) aus einem Halogenalkanol W-W' erhalten wurde, dessen OH-Funktion zuvor geschützt wurde, wobei das Halogenalkanol ausgewählt ist unter : 3-Chlor- oder 3-Brom-1-pentanol, 4-Chlor- oder 4-Brom-1-butanol, 5-Chlor- oder 5-Brom-1-pentanol, 6-Chlor- oder 6-Brom-1-hexanol und 7-Chlor- oder 7-Brom-1-heptanol.

3. Verfahren nach einem der Ansprüche 1 und 2, dadurch gekennzeichnet, daß die Schutzgruppe P der Verbindungen der Formel (II) eine Tetrahydropyranylgruppe, eine Tetrahydrofuranylgruppe, eine Trimethylsilylgruppe, eine Triäthylsilylgruppe, eine Triphenylmethylgruppe, eine Diphenylmethylgruppe, eine Benzylgruppe oder eine t-Butylgruppe ist.

4. Verfahren nach Anspruch 1, dadurch gekennzeichnet, daß die Verbindung der Formel (III) durch Acetylierung, Mesylierung oder Tosylierung eines 2E, 4E-Dienalkohols, ausgewählt unter : 2E, 4E-Hexadien-1-ol, 2E, 4E-Heptadien-1-ol, 2E, 4E-Octadien-1-ol und 2E, 4E-Nonadien-1-ol, erhalten wird.

5. Verfahren nach einem der vorhergehenden Ansprüche, dadurch gekennzeichnet, daß es sich beim Kupferkatalysator um Kupferjodid, Kupferchlorid oder Kupferbromid oder um Dilithiumtetrachlorocuprat handelt.

6. Verfahren nach einem der vorhergehenden Ansprüche, dadurch gekennzeichnet, daß die Menge an Katalysator zwischen 0,01 und 0,06 Mol liegt und vorzugsweise ungefähr 0,04 Mol pro Mol Verbindung der Formel (III) ausmacht.

7. Verfahren nach einem der vorhergehenden Ansprüche, dadurch gekennzeichnet, daß der Überschuß des Magnesiumderivats der Formel (II) mindestens 0,2 Mol, vorzugsweise zwischen 0,25 und 0,6 Mol, bezogen auf die molare Menge der Verbindung der Formel (III) beträgt.

8. Verfahren nach einem der vorhergehenden Ansprüche, dadurch gekennzeichnet, daß die Endkonzentration der Reaktionspartner im Tetrahydrofuran zwischen 0,1N und 0,8N, vorzugsweise bei ungefähr 0,5N, liegt.

9. Verfahren nach einem der vorhergehenden Ansprüche, dadurch gekennzeichnet, daß die Hydrolyse zur Freisetzung der Schutzgruppe in alkoholischem Milieu in Gegenwart von p-Toluolsulfonsäure durchgeführt wird.